(19) Europäisches Patentamt
European Patent Office
Office européen des brevets

(11) Veröffentlichungsnummer: **0 066 231**
**B1**

(12) # EUROPÄISCHE PATENTSCHRIFT

(45) Veröffentlichungstag der Patentschrift: **11.09.85**

(51) Int. Cl.[4]: **C 07 C 125/06**

(21) Anmeldenummer: **82104539.0**

(22) Anmeldetag: **25.05.82**

(54) **Verfahren zur Herstellung von N-substituierten Urethanen.**

(30) Priorität: **03.06.81 DE 3122013**

(43) Veröffentlichungstag der Anmeldung:
**08.12.82 Patentblatt 82/49**

(45) Bekanntmachung des Hinweises auf die Patenterteilung:
**11.09.85 Patentblatt 85/37**

(84) Benannte Vertragsstaaten:
**BE DE FR GB IT NL**

(56) Entgegenhaltungen:
**US - A - 2 677 698**

(73) Patentinhaber: **BAYER AG, Konzernverwaltung RP Patentabteilung, D-5090 Leverkusen 1 Bayerwerk (DE)**

(72) Erfinder: **Heitkämper, Peter, Dr., Fliederweg 14, D-4047 Dormagen 11 (DE)**
Erfinder: **König, Klaus, Dr., Heymannstrasse 50, D-5090 Leverkusen 1 (DE)**

**EP 0 066 231 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von N-substituierten Urethanen durch Umsetzung von primären Aminen mit N,N'-Diarylharnstoffen und hochsiedenden Monoalkoholen unter Abspaltung von destillativer Abtrennung von Arylaminen.

N-substituierte Urethane stellen wertvolle Zwischen- und Endprodukte dar, die zunehmend an Bedeutung gewinnen. Sie eignen sich beispielsweise als Pflanzenschutzmittel oder zur Herstellung von Isocyanaten.

N-substituierte Urethane werden üblicherweise durch Umsetzung der entsprechenden Isocyanate mit Alkoholen oder der entsprechenden Amine mit Chlorameisensäureestern hergestellt. Dabei werden die Isocyanate bzw. die Chlorameisensäureester mit Hilfe von Phosgen gewonnen, dessen Verwendung wegen der großen Giftigkeit erhebliche Sicherheitsmaßnahmen erfordert.

Weiterhin lassen sich N-substituierte Urethane durch Umsetzung geeigneter Stickstoffverbindungen mit Kohlenmonoxid in Gegenwart von Alkoholen herstellen. So sind beispielsweise in den deutschen Offenlegungsschriften 2 603 574 und 2 614 101 Verfahren zur Herstellung von Urethanen aus Nitroverbindungen durch Umsetzung mit Kohlenmonoxid und Alkoholen beschrieben. Nachteilig bei diesen Verfahren ist, daß Umsetzungen mit giftigem und brennbarem Kohlenmonoxid unter Druck durchgeführt werden müssen und deshalb dazu aufwendige Sicherheitsmaßnahmen notwendig sind. Außerdem erfordern solche Carbonylierungen den Einsatz von speziellen Katalysatorsystemen, wodurch die Verfahren mit erheblichen Kosten belastet sind.

In der US-Patentschrift 2 806 051 ist ein Verfahren zur Herstellung von N-substituierten Urethanen beschrieben, bei dem primäre Amine mit Harnstoff und Alkoholen umgesetzt werden, wobei entstehender Ammoniak aus dem Reaktionsgemisch entfernt wird. Wenn auch die Verwendung von Harnstoff als Ausgangsprodukt grundsätzlich einige Vorzüge enthält, so ist das Verfahren besonders für schwach basische Amine oder mehrfunktionelle Amine mit deutlichen Nachteilen behaftet. So werden bei diesen Umsetzungen auch N-unsubstituierte Carbamidsäureester sowie Cyanursäure, Polyurete und andere irreversible Zersetzungsprodukte des Harnstoffes als Nebenprodukte gebildet. Weitere Nebenprodukte dieser Umsetzungen sind N,N'-disubstituierte Harnstoffe, die beim Einsatz von mehrfunktionellen Aminen als schwer lösliche Oligo- oder Polyharnstoffe ausfallen und den Herstellungsprozeß stören können. Es ergeben sich Produktgemische, die gegebenenfalls aufwendig und kostspielig aufgearbeitet werden müssen, besonders, wenn die Umsätze nicht vollständig sind und die Gemische infolgedessen noch eine Restkonzentration an Amin aufweisen. Dabei können die gewonnenen Ausbeuten an gewünschtem Urethan mäßig sein. Die genannten Schwierigkeiten treten besonders beim Einsatz schwach basischer Amine oder mehrfunktioneller Amine auf.

Die genannten Nachteile werden auch nicht durch die in den deutschen Offenlegungsschriften 2 917 493 und 2 917 569 beschriebenen Verfahren beseitigt. Bei diesen Verfahren werden Di- und/oder Polyamine mit Harnstoff und Alkoholen umgesetzt.

Ähnliche Nachteile ergeben sich bei der Herstellung von N-substituierten Urethanen durch Umsetzung von primären Aminen mit N-unsubstituierten Carbamidsäureestern und gegebenenfalls Harnstoff und gegebenenfalls Alkoholen, wobei der entstehende Ammoniak aus dem Reaktionsgemisch entfernt wird. Dieses Herstellungsverfahren ist beispielsweise in den deutschen Offenlegungsschriften 2 917 490 und 2 917 568 sowie in den europäischen Patentschriften 0 018 581 und 0 018 583 beschrieben. Auch dabei entstehen die genannten irreversiblen Nebenprodukte. Außerdem reagieren N-unsubstituierte Carbamidsäureester im Vergleich zu Harnstoff deutlich langsamer, so daß besonders beim Einsatz schwach basischer Amine oder mehrfunktioneller Amine ein unvollständiger Umsatz resultiert.

Aufgabe der vorliegenden Erfindung war es nun, ein Verfahren zur Herstellung von N-substituierten Urethanen zur Verfügung zu stellen, das die genannten Nachteile nicht aufweist.

Diese Aufgabe konnte durch das nachstehend näher erläuterte erfindungsgemäße Verfahren gelöst werden.

Gegenstand der vorliegenden Erfindung ist ein Verfahren zur Herstellung von N-substituierten Urethanen der allgemeinen Formel

$$R^1(NH-CO-OR^2)_n,$$

in welcher

$R^1$ für einen gesättigten, unsubstituierten, aliphatischen Kohlenwasserstoffrest mit 4 bis 18 Kohlenstoffatomen, einen gesättigten, gegebenenfalls alkylsubstituierten und/oder Methylenbrücken aufweisenden cycloaliphatischen Kohlenwasserstoffrest mit insgesamt 6 bis 25 Kohlenstoffatomen oder einen gegebenenfalls alkyl- und/oder halogen-substituierten und/oder Methylenbrükken aufweisenden aromatischen Kohlenwasserstoffrest mit insgesamt 6 bis 25 Kohlenstoffatomen steht,

$R^2$ die Bedeutung eines gegebenenfalls inerte Substituenten und/oder Ethergruppen aufweisenden aliphatischen Kohlenwasserstoffrestes mit insgesamt 6 bis 18 Kohlenstoffatomen, eines gegebenenfalls inerte Substituenten aufweisenden cycloaliphatischen Kohlenwasserstoffrestes mit insgesamt 6 bis 15 Kohlenstoffatomen oder eines gegebenenfalls inerte Substituenten aufweisenden araliphatischen Kohlenwasserstoffrestes mit insgesamt 7 bis 18 Kohlenstoffatomen hat, und

n für 1, 2 oder 3 steht,

dadurch gekennzeichnet, daß man

a) primäre Amine der allgemeinen Formel

$R^1(NH_2)_n$,

in der $R^1$ und n die genannte Bedeutung haben, mit

b) Harnstoffen der allgemeinen Formel

$R^3-NH-CO-NH-R^3$,

in welcher $R^3$ die Bedeutung eines einem gegebenenfalls inerte Substituenten aufweisenden primären aromatischen Monoamin mit einem bei Normaldruck mindestens 10°C unterhalb des Siedepunktes des Amins $R^1(NH_2)_n$ liegenden Siedepunkt zugrundeliegenden, gegebenenfalls inerte Substituenten aufweisenden, aromatischen Kohlenwasserstoffrestes mit insgesamt 6 bis 14 Kohlenstoffatomen hat, und

c) primären oder sekundären Alkoholen, die bei Normaldruck einen mindestens 5°C höheren Siedepunkt als das Amin $R^3-NH_2$ besitzen und der allgemeinen Formel

$R^2-OH$

entsprechen, in der $R^2$ die genannte Bedeutung hat,

bei Temperaturen zwischen 150 und 300°C umsetzt und entstehendes Amin $R^3-NH_2$, gegebenenfalls im Gemisch mit Alkohol $R^2-OH$, während der Umsetzung destillativ aus dem Reaktionsgemisch entfernt.

Ausgangsmaterialien für das erfindungsgemäße Verfahren sind a) primäre Amine der allgemeinen Formel

$R^1(NH_2)_n$,

in der $R^1$ und n die bereits obengenannte Bedeutung haben.

Gemische von diesen Definitionen entsprechenden, primären Aminen können ebenfalls eingesetzt werden. Die Monoamine der genannten allgemeinen Formel müssen bei Normaldruck einen mindestens 10°C, vorzugsweise mindestens 25°C über dem Siedepunkt des dem nachstehend näher beschriebenen Ausgangsmaterial b) (disubstituierter Harnstoff) zugrundeliegenden Monoamins liegenden Siedepunkt aufweisen. Dies bedeutet insbesondere, daß im Falle der Verwendung von keine Substituenten aufweisenden aliphatischen oder cycloaliphatischen Monoaminen der genannten allgemeinen Formel nur solche in Betracht kommen können, für welche $R^1$ mindestens 9 Kohlenstoffatome aufweist. Im Falle der Verwendung von aromatischen Monoaminen der genannten allgemeinen Formel müssen diese selbstverständlich von dem dem Ausgangsmaterial b) zugrundeliegenden Monoamin verschieden sein und der genannten Bedingung bezüglich der Siedepunktdifferenz genügen.

Beispiele geeigneter primärer Amine a) sind

Decylamin, iso-Dodecylamin, n-Stearylamin, 2-(2-Butoxy-ethoxy)-ethylamin,
1-Butyl-4-methoxy-butylamin, 3-Butyl-cyclohexylamin, 4-Cyclohexyl-cyclohexylamin,
4-Chloranilin, 3,4-Dichloranilin, 3-Toluidin, 3-Chlor-4-methyl-anilin, 3,5-Dimethylanilin,
4-Cyclohexyl-anilin, 4-Benzylanilin, 1-Naphthylamin, 2-Naphthylamin, 2-Phenyl-ethylamin,
Tetramethylendiamin, Hexamethylendiamin, 2,2,4-Trimethyl-hexamethylendiamin,
Isophorondiamin, 1,4-Diamino-cyclohexan, 4,4'-Diamino-dicyclohexylmethan,
2,4-Diamino-1-methyl-cyclohexan, 2,6-Diamino-1-methyl-cyclohexan,
4-Aminocyclohexyl-4'-aminophenyl-methan, 1,3-Diaminobenzol, 2-Chlor-1,4-diaminobenzol,
2,4-Diaminotoluol, 2,6-Diaminotoluol, 2,4-Diamino-3,5-diethyl-toluol, 1,5-Diaminonaphthalin,
2,7-Diaminonaphthalin, 2,2'-Diamino-diphenylmethan, 2,4'-Diamino-diphenylmethan,
4,4'-Diamino-diphenylmethan, 3,3'-Dichlor-4,4'-diamino-diphenylmethan,
2,2-Bis-(4'-aminophenyl)-propan, 1,1-Bis-(4'-aminophenyl)-cyclohexan,
4,4',4''-Triamino-triphenylmethan, 1,3-Xylylendiamin, 1,4-Xylylendiamin,
3,4'-Diamino-4-methyl-diphenylmethan.

Die beispielhaft genannten Diamino-diphenylmethan-Isomeren können auch im Gemisch mit höherkernigen Homologen Verwendung finden, d. h. beispielsweise in Form der bekannten in Gegenwart von sauren Katalysatoren erhaltenen Anilin-Formaldehyd-Kondensate, die als »Polyamingemische der Diphenylmethanreihe« bezeichnet werden können.

Weitere Ausgangsmaterialien für das erfindungsgemäße Verfahren sind b) N,N'-Diarylharnstoffe der allgemeinen Formel

$R^3-NH-CO-NH-R^3$,

in der $R^3$ die bereits obengenannte Bedeutung hat. Beispiele geeigneter Diarylharnstoffe sind

N,N'-Diphenylharnstoff, N,N'-Di-(2-tolyl)-harnstoff, N,N'-Di-(3-tolyl)-harnstoff, N,N'-Di-(4-tolyl)-harnstoff, N,N'-Di-(3-ethylphenyl)-harnstoff, N,N'-Di-(4-ethylphenyl)-harnstoff, N,N'-Di-(3-methoxyphenyl)-harnstoff, N,N'-Di-(4-methoxyphenyl)-harnstoff.

Bevorzugt wird jedoch für das erfindungsgemäße Verfahren N,N'-Diphenylharnstoff verwendet.

Schließlich werden als Ausgangsmaterialien c) für das erfindungsgemäße Verfahren primäre oder sekundäre, vorzugsweise primäre Monoalkohole eingesetzt, deren Siedepunkt bei Normaldruck mindestens 5°C, vorzugsweise mindestens 20°C oberhalb des Siedepunktes des dem Diarylharnstoff $R^3-NH-CO-NH-R^3$ zugrundeliegenden Amins $R^3-NH_2$ liegt und die der allgemeinen Formel

$R^2-OH$

entsprechen, in der $R^2$ die bereits oben angegebene Bedeutung hat.

Vorzugsweise werden solche Alkohole der genannten allgemeinen Formel eingesetzt, für welche $R^2$ für einen gesättigten, gegebenenfalls Etherbrücken aufweisenden primären aliphatischen Kohlenwasserstoffrest mit 6 bis 18 Kohlenstoffatomen steht.

Beispiele geeigneter Alkohole c) sind geradkettig oder verzweigtes Octanol, Nonanol, Decanol, Undecanol, Dodecanol, Tetradecanol, Hexadecanol oder Octadecanol sowie 2-(2-Ethoxy-ethoxy)-ethanol, 2-(2-Butoxy-ethoxy)-ethanol, 2-Phenylethanol, 2-Phenoxyethanol, 2-(2-Phenoxy-ethoxy)-ethanol, Cyclooctanol, Benzylalkohol, 2,4,5-Trimethyl-cyclohexanol, 1,2,3,4-Tetrahydronaphthol-(2), Perhydro-naphthol-(2). Selbstverständlich können beim erfindungsgemäßen Verfahren auch Gemische von Alkoholen $R^2-OH$ eingesetzt werden.

Die für das erfindungsgemäße Verfahren verwendeten Diarylharnstoffe $R^3-NH-CO-NH-R^3$ werden nach bekannten Verfahren der Technik hergestellt, beispielsweise durch Umsetzung von aromatischen Aminen mit Kohlendioxid entsprechend der britischen Patentschrift 622 955.

Auf einfache Weise und mit hohen Ausbeuten lassen sich die Diarylharnstoffe durch Umsetzung entsprechender Arylamine $R^3-NH_2$ mit Harnstoff unter Abtrennung von Ammoniak herstellen (vgl. auch: Liebigs Ann. Chem., 131, 251 [1864]). Selbstverständlich können die für das erfindungsgemäße Verfahren verwendeten Diarylharnstoffe auch nach einer beliebigen anderen Methode hergestellt werden.

Es ist für das erfindungsgemäße Verfahren nicht erforderlich, daß die eingesetzten Diarylharnstoffe $R^3-NH-CO-NH-R^3$ eine hochreine Qualität besitzen. Insbesondere Beimengungen von entsprechendem Arylamin $R^3-NH_2$ zum Diarylharnstoff stören die erfindungsgemäße Umsetzung nicht, da dabei ohnehin Arylamin $R^3-NH_2$ freigesetzt und destillativ aus dem Reaktionsgemisch entfernt wird.

So besteht eine besondere Ausführungsform des erfindungsgemäßen Verfahrens darin, daß man rohe Diarylharnstoffe $R^3-NH-CO-NH-R^3$ einsetzt, die man in einer vorgeschalteten Umsetzung dadurch erhält, daß man Harnstoff mit Arylamin $R^2-NH_2$ im Molverhältnis 1 : 1,8 bis 1 : 10, vorzugsweise 1 : 2 bis 1 : 4 bei Temperaturen zwischen 130 und 250°C, vorzugsweise zwischen 150 und 210°C unter Abspaltung und Abtrennung von Ammoniak umsetzt. Aus diesen nach Beendigung der Ammoniakabspaltung vorliegenden rohen Diarylharnstoffen kann gewünschtenfalls vor dem Einsatz in das erfindungsgemäße Verfahren überschüssiges Arylamin $R^3-NH_2$ ganz oder teilweise abgetrennt werden, beispielsweise durch Destillation. Die rohen Diarylharnstoffe können aber auch ohne weitere Behandlung in das erfindungsgemäße Verfahren eingesetzt werden.

Bei der Durchführung des erfindungsgemäßen Verfahrens kommen die Reaktionspartner in Mengen zum Einsatz, die einem Molverhältnis von Aminogruppen des Amins $R^1(NH_2)_n$ zu Diarylharnstoff $R^3-NH-CO-NH-R^3$ von insgesamt 1 : 1 bis 1 : 5, vorzugsweise 1 : 1 bis 1 : 1,5, besonders bevorzugt 1 : 1 bis 1 : 1,05, bzw. einem Molverhältnis von Aminogruppen des Amins $R^1(NH_2)_n$ zu Alkohol $R^2-OH$ von insgesamt 1 : 1 bis 1 : 20, vorzugsweise 1 : 1,5 bis 1 : 10 entsprechen.

Die erfindungsgemäße Umsetzung wird in einem Temperaturbereich von 150 bis 300°C, vorzugsweise 180 bis 250°C, durchgeführt.

Der Druck wird bei der Durchführung des erfindungsgemäßen Verfahrens so eingestellt, daß das bei der Umsetzung entstehende und/oder gegebenenfalls bereits im Reaktionsgemisch befindliche Arylamin $R^3-NH_2$ gegebenenfalls im Gemisch mit Alkohol $R^2-OH$ siedet und durch Destillation entfernt werden kann. Der Druck beträgt im allgemeinen 0,001 bis 1,5 bar, vorzugsweise 0,01 bis

1,1 bar.

Die erfindungsgemäße Umsetzung ist im allgemeinen nach einer Reaktionszeit von 1 bis 20 Stunden, vorzugsweise 2 bis 10 Stunden, insbesondere 3 bis 6 Stunden, beendet.

Bei der Durchführung des erfindungsgemäßen Verfahrens können die Umsetzungen durch Mitverwendung von Katalysatoren beschleunigt werden. Geeignete Katalysatoren sind beispielsweise Lewis-Säuren wie $BF_3$, $BCl_3$, $B(OC_2H_5)_3$, $B(OC_4H_9)_3$, $AlCl_3$, $AlBr_3$, $SnCl_4$, Dibutylzinnoxid, $SbCl_5$, $TiCl_4$, $TiBr_4$, $FeCl_3$, Kobaltoctoat, $ZnCl_2$, Zinkoctoat, CuCl und/oder Salze und Komplexverbindungen von Übergangsmetallen, soweit sie nicht bereits in die Gruppe der Lewis-Säuren fallen, wie Eisencarbonyle, Acetylacetonate von Eisen, Nickel, Kobalt, Zink, Mangan, Molybdän, Titan, Thorium, Vanadium oder Zirkonium, Bis-(dibenzoylmethan)-kupfer. Selbstverständlich können auch Gemische mehrerer derartiger Verbindungen als Katalysator verwendet werden.

Als Katalysator besonders gut geeignete Verbindungen sind beispielsweise Zinkchlorid, Zinkacetat, Zinkoctoat, Zinkoxid, Zinndichlorid, Zinntetrachlorid, Dibutylzinnoxid, Dibutylzinndiethylat, Dimethylzinndichlorid, Dibutylzinndilaurat, Kobalttrichlorid, Kobalttriacetat, Kupferchlorid oder Kupferacetat.

Der Katalysator wird, falls überhaupt, im allgemeinen in einer Menge von 0,001 bis 5 Gew.-%, vorzugsweise 0,01 bis 2 Gew.-%, bezogen auf das Gesamtgewicht der Ausgangsmaterialien, dem Reaktionsgemisch zugesetzt. Man wird in der Praxis selbstverständlich bestrebt sein, die Konzentration an Katalysator möglichst gering zu halten. Die optimale Konzentration hängt von der Art der Ausgangsmaterialien und der Wirksamkeit des verwendeten Katalysators ab und kann in einem einfachen Vorversuch ermittelt werden.

Bei der Durchführung des erfindungsgemäßen Verfahrens kann die Umsetzung so durchgeführt werden, daß die Ausgangsmaterialien Amin $R^1(NH_2)_n$, Diarylharnstoff $R^3-NH-CO-NH-R^3$ und Alkohol $R^2-OH$ gemischt und in einer Destillationsapparatur unter dem notwendigen Druck erhitzt werden, wobei entstehendes und gegebenenfalls bereits im Reaktionsgemisch befindliches Arylamin $R^3-NH_2$ durch Destillation aus dem Reaktionsgemisch entfernt wird.

Es kann bei den erfindungsgemäßen Umsetzungen von Vorteil sein, zunächst den einzusetzenden Diarylharnstoff $R^3-NH-CO-NH-R^3$ mit dem einzusetzenden Alkohol $R^2-OH$ ohne das einzusetzende Amin $R^1(NH_2)_n$ oder nur mit einem Teil des einzusetzenden Amins $R^1(NH_2)_n$ in einer Destillationsapparatur unter dem erforderlichen Druck auf die gewünschte Temperatur zu bringen und dann das Amin $R^1(NH_2)_n$ bzw. den Rest des Amins $R^1(NH_2)_n$ gegebenenfalls im Gemisch mit Alkohol $R^2-OH$ in dem Maße in das Reaktionsgemisch einzudosieren, wie es dem Grad der Reaktion entspricht. Auf diese Weise kann vermieden werden, daß im Reaktionsgemisch unerwünscht hohe Konzentrationen an Amin $R^1(NH_2)_n$ vorliegen. Dieses kann insbesondere bei solchen mehrfunktionellen Aminen $R^1(NH_2)_n$ von Bedeutung sein, die im Reaktionsgemisch schwer- oder unlösliche Polyharnstoffe bilden.

Durch die destillative Entfernung des gebildeten Arylamins $R^3-NH_2$ aus dem Reaktionsgemisch während der erfindungsgemäßen Umsetzung wird erreicht, daß sich das Reaktionsgleichgewicht in Richtung auf das angestrebte Verfahrensprodukt verschiebt, wodurch ein quantitativer Umsatz des Amins $R^1(NH_2)_n$ gewährleistet ist.

Die destillative Abtrennung von Arylamin $R^3-NH_2$ aus dem Reaktionsgemisch erfolgt nach bekannten Methoden der Technik, beispielsweise durch Abstreifdestillation mit Hilfe eines auf das Reaktionsgefäß aufgesetzten Kondensators. Im allgemeinen ist es jedoch zweckmäßig, die aus dem Reaktionsgefäß entweichenden Dämpfe mit Hilfe eins Dephlegmators oder insbesondere mit Hilfe einer trennwirksamen Kolonne zu fraktionieren. Auf diese Weise kann verhindert werden, daß Anteile von Amin $R^1(NH_2)_n$ oder unerwünscht große Mengen an Alkohol $R^2-OH$ zusammen mit dem Arylamin $R^3-NH_2$ destillativ aus dem Reaktionsgemisch entfernt werden.

Es ist für das erfindungsgemäße Verfahren nicht wesentlich, daß das aus dem Reaktionsgemisch destillativ abgetrennte Arylamin $R^3-NH_2$ frei von Alkohol $R^2-OH$ ist. Es kann sogar von Vorteil sein, besonders gegen Ende der erfindungsgemäßen Umsetzung, wenn Anteile von Alkohol $R^2-OH$ zusammen mit dem Arylamin $R^3-NH_2$ aus dem Reaktionsgemisch entfernt werden. Es muß dabei nur gewährleistet sein, daß im Reaktionsgemisch für die Umsetzung ausreichende Mengen an Alkohol $R^2-OH$ verbleiben.

Das erfindungsgemäße Verfahren kann diskontinuierlich oder kontinuierlich durchgeführt werden. Bei der kontinuierlichen Durchführung des erfindungsgemäßen Verfahrens wird zweckmäßig eine Serie von kaskadenartig verbundenen Reaktionsgefäßen als Apparatur verwendet. Dabei kann es vorteilhaft sein, die Reaktionstemperatur, den Reaktionsdruck und/oder die mittlere Verweilzeit in den einzelnen Reaktionsgefäßen unterschiedlich zu wählen. Die jeweils aus den Reaktionsgefäßen entweichenden Dämpfe können dabei einzeln, beispielsweise in jeweils auf die Reaktionsgefäße aufgesetzten Kolonnen, oder nach Vermischung gemeinsam fraktioniert werden, um die erfindungsgemäße Abtrennung von Arylamin $R^3-NH_2$ zu bewerkstelligen.

Selbstverständlich kann bei der kontinuierlichen Durchführung des erfindungsgemäßen Verfahrens auch eine beliebige andere Apparatur verwendet werden.

Nach Beendigung der erfindungsgemäßen Umsetzung kann gegebenenfalls noch im Reaktionsgemisch vorliegender überschüssiger Alkohol $R^2-OH$ durch Destillation, vorzugsweise unter vermindertem Druck vom Verfahrensprodukt, getrennt werden.

Beim erfindungsgemäßen Verfahren entstehen als Verfahrensprodukte N-monosubstituierte O-Alkylurethane der Formel

$$R^1(NH-CO-OR^2)_n,$$

in der $R^1$, $R^2$ und n die bereits obengenannte Bedeutung haben. Das erfindungsgemäße Verfahren verläuft nach folgender Reaktionsgleichung:

$$R^1(NH_2)_n + n\,R^3-NH-CO-NH-R^3 + n\,R^2-OH \rightarrow R^1(NH-CO-OR^2)_n + 2\,n\,R^3-NH_2$$

Es muß als äußerst überraschend bezeichnet werden, daß beim erfindungsgemäßen Verfahren die Bildung von substituierten Harnstoffen, denen das eingesetzte Amin $R^1(NH_2)_n$ zugrunde liegt, insbesondere beim Einsatz von mehrfunktionellen Aminen die Bildung von Polyharnstoffen praktisch vollständig unterbleibt. Da die Umsetzung bei Temperaturen erfolgt, bei denen normalerweise O-Alkylurethane in Isocyanate und Alkohole gespalten werden, mußte damit gerechnet werden, daß sich unter diesen Bedingungen Isocyanate der Formel

$$R^1(NH-CO-OR^2)_{n-m}(NCO)_m,$$

in der $R^1$, $R^2$ und n die bereits obengenannte Bedeutung haben und m = n ist und die Bedeutung einer positiven ganzen Zahl hat, im Reaktionsgemisch bilden und mit der Aminkomponente $R^1(NH_2)_n$ zu unerwünschten Harnstoffen vereinigen. Diese Nebenreaktion wird jedoch praktisch nicht beobachtet.

Die erfindungsgemäß hergestellten Verfahrensprodukte stellen Carbamidsäureester dar, denen notwendigerweise verhältnismäßig hochsiedende Alkohole zugrundeliegen. Sollten jedoch Carbamidsäureester auf der Basis niedriger siedender Alkohole gewünscht werden, wie es beispielsweise bei der Herstellung von Isocyanaten durch thermische Spaltung von O-Alkylurethanen der Fall sein kann, so können die Verfahrensprodukte durch Umsatz mit dem entsprechenden niedriger siedenden Alkohol in einer Austauschreaktion zu den gewünschten Produkten umurethanisiert werden. Dieses Verfahren ist beispielsweise in der deutschen Offenlegungsschrift 2 943 549 beschrieben.

Durch die nachfolgenden Beispiele wird das erfindungsgemäße Verfahren näher erläutert.

## Beispiel 1

In einem 2-l-Rundkolben mit aufgesetzter Füllkörperkolonne wurden 236 g n-Decylamin, 320 g N,N'-Diphenylharnstoff und 900 g 2-Phenyl-ethanol vorgelegt und unter kräftigem Rühren auf 210°C erhitzt. Das Gemisch wurde 4 Stunden lang bei 210°C und Normaldruck gerührt, wobei Anilin über die Kolonne abdestilliert wurde. Anschließend wurde die Reaktionstemperatur auf 230°C gesteigert und 15 Minuten lang Phenylethanol abdestilliert. Das Rohgemisch wurde dann durch Vakuumdestillation (zunächst bei 20 mbar, schließlich bei 1 mbar) von dem größten Teil des Phenylethanol-Überschusses befreit. Es blieben 472 g Rohprodukt zurück, das mittels Flüssigkeitschromatographie (HPLC) analysiert wurde. Das Rohprodukt bestand zu 96,1 Gew.-% aus N-Decyl-O-(2-phenyl-ethyl)-urethan (Ausbeute: 99% der Theorie). Im Destillat wurden insgesamt 279 g Anilin nachgewiesen.

## Beispiel 2

In einem 2-l-Rundkolben mit aufgesetztem Rückflußkühler wurden 420 g Anilin und 92 g Harnstoff vorgelegt und unter Rühren innerhalb von 30 Minuten auf 190°C erhitzt. Dabei wurde Ammoniak-Gas freigesetzt, das über den Rückflußkühler abgeleitet und in einem Wäscher in Wasser absorbiert wurde. Nach weiteren 1,5 Stunden, in deren Verlauf die Reaktionstemperatur auf 200°C gesteigert wurde, war die Ammoniak-Abspaltung beendet. Die Apparatur wurde kurz mit Stickstoff gespült, um Reste von Ammoniak-Gas zu entfernen. Dann wurde der Rückflußkühler gegen eine Destillationsbrükke ausgewechselt. In das Reaktionsgemisch wurde dann unter kräftigem Rühren eine 190°C heiße Lösung von 404 g n-Stearylamin in 800 g n-Dodecanol eingemischt. Das Gemisch wurde auf 230°C erhitzt und bei dieser Temperatur unter Normaldruck 5 Stunden lang gerührt, wobei eine Mischung von 417 g Anilin und 77 g Dodecanol überdestillierte. Anschließend wurde der größte Teil des überschüssigen Dodecylalkohols im Vakuum abdestilliert, wobei 774 g Rohprodukt zurückblieben. Vom Rohprodukt wurden 5,18 g mittels Säulenchromatographie an Kieselgel aufgetrennt. Es wurden dabei 4,73 g N-Octadecyl-O-dodecyl-urethan isoliert. Das entsprach einer Ausbeute von 98% der Theorie.

Beispiel 3

In einem 2-l-Rundkolben mit aufgesetzter Füllkörperkolonne wurden 163 g 2-[2-(2-Methoxy-ethoxy)-ethoxy]-ethylamin, 213 g N,N'-Diphenylharnstoff und 700 g 2-Phenoxy-ethanol vorgelegt. Unter kräftigem Rühren wurde das Gemisch bei Normaldruck auf 220° C erhitzt, wobei Anilin freigesetzt und über die Kolonne abdestilliert wurde. Die Reaktionstemperatur wurde im Verlauf von 4 Stunden auf 233° C gesteigert, während weiterhin freigesetztes Anilin abdestilliert wurde. Anschließend wurde aus dem Reaktionsgemisch der größte Teil des überschüssigen Alkohols durch Vakuumdestillation (zunächst bei 20 mbar, dann bei 1 mbar) abgetrennt, wobei 333 g flüssiges Rohprodukt zurückblieben. Das Rohprodukt enthielt nach flüssigkeitschromatographischer Analyse (HPLC) 96,6 Gew.-% N--2-[2-(2-Methoxy-ethoxy)-ethoxy]-ethyl-O-(2-phenoxy-ethyl)-urethan (Ausbeute: 98% der Theorie). Im Destillat wurden insgesamt 186 g Anilin nachgewiesen.

Beispiel 4

In einem 2-l-Rundkolben mit aufgesetzter Füllkörperkolonne wurden 320 g Anilin und 73 g Harnstoff vorgelegt und unter Rühren innerhalb von 30 Minuten auf 190° C erhitzt. Dabei wurde Ammoniakgas freigesetzt, das über die Kolonne abgeleitet und in Wasser absorbiert wurde. Nach weiteren 1,5 Stunden, in deren Verlauf die Reaktionstemperatur auf 200° C gesteigert wurde, war die Ammoniak-Abspaltung beendet. Die Apparatur wurde kurz mit Stickstoff gespült. Das Reaktionsgemisch wurde dann unter Rühren mit einer 190° C heißen Lösung von 170 g 3-Chlor-4-methyl-anilin in 900 g n-Decanol versetzt. Das Gemisch wurde anschließend 5 Stunden lang bei 225° C und Normaldruck gerührt, wobei Anilin über die Kolonne abdestilliert wurde. Anschließend wurde der Druck langsam auf 500 mbar verringert, bis reiner Decylalkohol überdestillierte. Aus dem Reaktionsgemisch wurde schließlich der größte Teil des Decanol-Überschusses bei zunächst 20 mbar, dann bei 1 mbar, abdestilliert, wobei 370 g Rohprodukt zurückblieben. Laut flüssigkeitschromatographischer Analyse (HPLC) enthielt das Rohprodukt 95,7 Gew.-% N-(3-Chlor-4-methyl-phenyl)-O-decyl-urethan (Ausbeute: 98% der Theorie). Im Destillat wurden insgesamt 318 g Anilin nachgewiesen.

Beispiel 5

In einem 2-l-Rundkolben mit aufgesetzter Füllkörperkolonne wurde ein Gemisch von 195 g 3,4-Dichloranilin, 257 g N,N'-Diphenylharnstoff und 1000 g 2-Phenoxyethanol unter kräftigem Rühren auf 230° C erhitzt. Innerhalb von 6 Stunden wurde bei 230° C und Normaldruck Anilin abdestilliert. Dann wurde der Druck auf 500 mbar gesenkt und weiter destilliert, bis reines Phenoxyethanol überging. Anschließend wurde der größte Teil des Phenoxyethanol-Überschusses durch Vakuumdestillation abgetrennt, wobei 407 g Rohprodukt zurückblieben. Dieses enthielt nach flüsigkeitschromatographischer Analyse (HPLC) 93,8 Gew.-% N-(3,4-Dichlorphenyl)-O-(2-phenoxy-ethyl)-urethan (Ausbeute: 97% der Theorie). Das Rohprodukt wurde aus Ligroin umkristallisiert und ergab farblose Kristalle vom Schmelzpunkt 79 bis 81° C. Im Destillat wurden insgesamt 222 g Anilin nachgewiesen.

Beispiel 6

In einem 2-l-Rundkolben, der mit einer Füllkörperkolonne und einem beheizbaren Tropftrichter versehen war, wurden 470 g Anilin und 122 g Harnstoff innerhalb von 30 Minuten unter kräftigem Rühren auf 190° C erhitzt. Dabei wurde Ammoniakgas freigesetzt, das über die Kolonne entwich und in Wasser absorbiert wurde. Nach weiteren 1,5 Stunden, in deren Verlauf die Temperatur auf 204° C gesteigert wurde, war die Ammoniak-Abspaltung beendet. Die Apparatur wurde kurz mit Stickstoff gespült. Dann wurde das Reaktionsgemisch mit 800 g 200° C heißem Decanol und 1 g Dibutylzinnoxid versetzt und auf 220° C erhitzt. In die 220° C heiße Mischung wurde unter Rühren bei Normaldruck innerhalb von 4 Stunden eine 50° C warme Lösung von 116 g Hexamethylendiamin in 400 g n-Decanol eingetropft, während Anilin über die Kolonne abdestilliert wurde. Nach Beendigung der Zugabe wurde das Reaktionsgemisch noch eine weitere Stunde bei 220° C und Normaldruck unter Anilin-Destillation gerührt. Anschließend wurde der Druck auf 500 mbar gesenkt, bis reines Decanol überdestillierte. Schließlich wurde der größte Teil des Decanol-Überschusses durch Vakuumdestillation abgetrennt, wobei 502 g Rohprodukt zurückblieben. Von dem Rohprodukt wurden 4,93 g mittels Säulenchromatographie an Kieselgel aufgetrennt. Dabei wurden 4,69 g Hexamethylen-bis-carbamidsäure-bis-decylester als Kristalle vom Schmelzpunkt 113° C erhalten (Ausbeute: 99% der Theorie). Im Destillat wurden insgesamt 467 g Anilin nachgewiesen.

Beispiel 7

In einem 2-l-Rundkolben mit Tropftrichter und Füllkörperkolonne wurde ein Gemisch von 427 g N,N'-Diphenylharnstoff, 1000 g 2-Phenoxy-ethanol und 1,5 g Dibutylzinnoxid unter Rühren auf 215°C erhitzt. Nach Einstellen eines Drucks von 600 mbar wurde im Verlaufe von 4 Stunden eine Lösung von 116 g Hexamethylendiamin in 400 g 2-Phenoxy-ethanol in das 215°C heiße, homogen flüssige Reaktionsgemisch eingetropft, während Anilin über die Kolonne abdestilliert wurde. Nach Beendigung der Zugabe wurde noch eine weitere Stunde bei 215°C und 600 mbar Anilin abdestilliert. Anschließend wurde der Druck langsam verringert, bis reines Phenoxyethanol überdestillierte. Schließlich wurde durch Vakuumdestillation der größte Teil des Phenoxyethanol-Überschusses abgetrennt, wobei 465 g Rohprodukt zurückblieben. Das Rohprodukt bestand nach flüssigkeitschromatographischer Analyse (HPLC) zu 93,6 Gew.-% aus Hexamethylen-bis-carbamidsäure-bis-(2-phenoxy-ethyl)-ester (Ausbeute: 98% der Theorie). Umkristallisation des Rohproduktes aus 1,2-Dichlorethan ergab farblose Kristalle vom Schmelzpunkt 152°C. Im Destillat wurden insgesamt 370 g Anilin nachgewiesen.

Beispiel 8

In einem 2-l-Rundkolben mit Tropftrichter und Füllkörperkolonne wurde ein Gemisch von 426 g N,N'-Diphenylharnstoff und 1000 g 2-Phenoxy-ethanol unter kräftigem Rühren auf 230°C erhitzt. In das 230°C heiße Gemisch wurde bei Normaldruck innerhalb von 4 Stunden eine Lösung von 210 g trans,trans-4,4'-Diamino-dicyclohexylmethan in 400 g 2-Phenoxyethanol eingetropft, während Anilin über die Kolonne abdestilliert wurde. Nach beendeter Zugabe wurde noch 1,5 Stunden lang das Gemisch unter weiterer Anilin-Destillation bei 230°C gerührt. Anschließend wurde der Druck vermindert, bis reines Phenoxyethanol überging. Schließlich wurde aus dem Reaktionsgemisch der größte Teil des Phenoxyethanol-Überschusses durch Vakuumdestillation abgetrennt, wobei 566 g Rohprodukt zurückblieben. Das Rohprodukt enthielt nach flüssigkeitschromatographischer Analyse (HPLC) 94,1 Gew.-% Dicyclohexylmethan-4,4'-bis-carbamidsäure-bis-(2-phenoxy-ethyl)-ester (Ausbeute: 99% der Theorie). Im Destillat wurden insgesamt 373 g Anilin nachgewiesen.

Beispiel 9

In einem 2-l-Rundkolben mit aufgesetzter Füllkörperkolonne wurde ein Gemisch von 426 g N,N'-Diphenylharnstoff, 122 g 2,4-Diamino-toluol, 1300 g 2-Phenoxy-ethanol und 2 g Dibutylzinndilaurat unter kräftigem Rühren auf 230°C erhitzt. Das Reaktionsgemisch wurde 6 Stunden lang bei 230°C und Normaldruck gerührt, während Anilin über die Kolonne abdestilliert wurde. Anschließend wurde der Druck innerhalb von 30 Minuten gesenkt, bis reines Phenoxy-ethanol überdestillierte. Aus dem Reaktionsgemisch wurde schließlich der größte Teil des Phenoxyethanol-Überschusses durch Vakuumdestillation abgetrennt, wobei 455 g Rohprodukt zurückblieben. Das Rohprodukt enthielt nach flüssigkeitschromatographischer Analyse (HPLC) 94,7 Gew.-% Toluol-2,4-bis-carbamidsäure-bis-(2-phenoxy-ethyl)-ester (Ausbeute: 96% der Theorie). Nach dem Umkristallisieren aus Ethanol ergab das Rohprodukt fast farblose Kristalle vom Schmelzpunkt 137°C. Im Destillat wurden insgesamt 364 g Anilin nachgewiesen.

Beispiel 10

In einem 2-l-Rundkolben mit Tropftrichter und Füllkörperkolonne wurde ein Gemisch von 430 g 3-Toluidin und 97 g Harnstoff innerhalb von 30 Minuten unter kräftigem Rühren auf 210°C erhitzt. Dabei wurde Ammoniakgas freigesetzt, das über die Kolonne abgeleitet und in Wasser absorbiert wurde. Nach weiteren 1,5 Stunden Reaktion bei 210°C war die Ammoniak-Abspaltung beendet. Die Apparatur wurde kurz mit Stickstoff gespült. Dann wurde das Reaktionsgemisch mit 1000 g 200°C heißem n-Dodecanol und 2 g Dimethylzinndichlorid versetzt und auf 230°C erhitzt. Nach Einstellen eines Druckes von 500 mbar wurde in das 230°C heiße Reaktionsgemisch im Verlaufe von 4 Stunden eine Lösung von 98 g 2,4-Diamino-toluol in 350 g Dodecanol eingetropft, während über die Kolonne 3-Toluidin abdestilliert wurde. Nach Beendigung der Zugabe wurde das Gemisch noch weitere 2 Stunden bei 230°C und 500 mbar unter Toluidin-Destillation gerührt. Dann wurde der Druck langsam gesenkt, bis reines Dodecanol überdestillierte. Schließlich wurde der größte Teil des Dodecanol-Überschusses durch Vakuumdestillation aus dem Reaktionsgemisch abgetrennt, wobei 475 g Rohprodukt zurückblieben. Das Rohprodukt enthielt nach flüssigkeitschromatographischer Analyse (HPLC) 90,6 Gew.-% Toluol-2,4-bis-carbamidsäure-bis-dodecylester (Ausbeute: 98% der Theorie). Das Rohprodukt wurde aus Ligroin umkristallisiert und ergab fast farblose Kristalle vom Schmelzpunkt 82°C. Im Destillat wurden insgesamt 425 g 3-Toluidin nachgewiesen.

Beispiel 11

In einem 2-l-Rundkolben mit Tropftrichter und Füllkörperkolonne wurde ein Gemisch von 330 g Anilin und 86 g Harnstoff unter Rühren innerhalb von 30 Minuten auf 190°C erhitzt. Dabei wurde Ammoniakgas freigesetzt, das über die Kolonne abgeleitet und in Wasser absorbiert wurde. Nach weiteren 1,5 Stunden, in deren Verlauf die Temperatur auf 205°C gesteigert wurde, war die Ammoniak-Abspaltung beendet. Die Apparatur wurde kurz mit Stickstoff gespült. Dann wurde die Reaktionsmischung mit 800 g 200°C heißem Decanol versetzt und auf 220°C erhitzt. Anschließend wurde unter Rühren bei Normaldruck im Laufe von 4 Stunden eine Lösung von 140 g 4,4'-Diamino-diphenylmethan in 300 g n-Decanol in das 220°C heiße Reaktionsgemisch eingetropft, während über die Kolonne Anilin abdestilliert wurde. Nach beendeter Zugabe wurde noch eine weitere Stunde bei 220°C und Normaldruck unter Anilin-Destillation gerührt. Dann wurde der Druck langsam verringert, bis reines Decanol überdestillierte. Schließlich wurde aus dem Reaktionsgemisch der größte Teil des Decanol-Überschusses durch Vakuumdestillation abgetrennt, wobei 407 g Rohprodukt zurückblieben. Das Rohprodukt enthielt nach flüssigkeitschromatographischer Analyse (HPLC) 96,2 Gew.-% Diphenylmethan-4,4'-bis-carbamidsäure-bis-decylester (Ausbeute: 98% der Theorie). Das Rohprodukt ergab nach dem Umkristallisieren aus Ethanol farblose Kristalle vom Schmelzpunkt 116°C. Im Destillat wurden insgesamt 327 g Anilin nachgewiesen.

Beispiel 12

In einem 2-l-Rundkolben mit aufgesetzter Füllkörperkolonne wurde ein Gemisch von 323 g N,N'-Diphenylharnstoff, 150 g 4,4'-Diamino-diphenylmethan, 1200 g 2-(2-Butoxy-ethoxy)-ethanol und 1 g Dibutylzinnoxid unter kräftigem Rühren auf 210 bis 212°C erhitzt und 6,5 Stunden lang bei 212°C und Normaldruck gerührt, während Anilin über die Kolonne abdestilliert wurde. Anschließend wurde die Temperatur auf 230°C gesteigert, und es wurde 30 Minuten lang weiter destilliert. Schließlich wurde der größte Teil des Butoxyethoxyethanol-Überschusses durch Vakuumdestillation aus dem Reaktionsgemisch abgetrennt, wobei 432 g Rohprodukt zurückblieben. Das Rohprodukt enthielt nach flüssigkeitschromatographischer Analyse (HPLC) 95,9 Gew.-% Diphenylmethan-4,4'-bis-carbamidsäure-bis-[(2-butoxy-ethoxy)-ethyl]-ester (Ausbeute: 95% der Theorie). Im Destillat wurden insgesamt 275 g Anilin nachgewiesen.

Beispiel 13

In einem 2-l-Rundkolben mit Tropftrichter und Füllkörperkolonne wurde ein Gemisch von 350 g Anilin und 85 g Harnstoff unter Rühren innerhalb von 30 Minuten auf 190°C erhitzt. Dabei wurde Ammoniakgas freigesetzt, das über die Kolonne abgeleitet und in Wasser absorbiert wurde. Nach weiteren 1,5 Stunden, in deren Verlauf die Temperatur auf 205°C gesteigert wurde, war die Ammoniak-Abspaltung beendet. Die Apparatur wurde kurz mit Stickstoff gespült. Dann wurde das Reaktionsgemisch mit 1000 g 200°C heißem 2-Phenoxyethanol versetzt und auf 230°C erhitzt. Unter kräftigem Rühren wurden anschließend 140 g eines handelsüblichen Gemisches aus 4,4'-, 2,4'-, 2,2'-Diamino-diphenylmethan und Polyphenyl-polymethylen-polyamin mit einem analysierten Äquivalentgewicht von 99,9 (bezogen auf Aminogruppen) innerhalb von 4 Stunden bei Normaldruck in das 230°C heiße Reaktionsgemisch eingetropft, während Anilin über die Kolonne abdestilliert wurde. Nach Beendigung der Zugabe wurde das Gemisch noch weitere 2 Stunden bei 230 bis 235°C und Normaldruck unter Anilin-Destillation gerührt. Dann wurde der Druck verringert, bis reines Phenoxyethanol überdestillierte. Schließlich wurde der größte Teil des Phenoxyethanol-Überschusses durch Vakuumdestillation aus dem Reaktionsgemisch abgetrennt, wobei 387 g Rohprodukt zurückblieben, die nach titrimetrischer Anaylse ($HClO_4$ in Eisessig) insgesamt 0,015 Mol Aminogruppen enthielten; das entsprach einem Aminogruppen-Umsatz von 99%. Der Restgehalt an 2-Phenoxy-ethanol im Rohprodukt wurde durch säulenchromatographische Abtrennung an Kieselgel zu 3,7 Gew.-% bestimmt. Das Rohprodukt stellte im wesentlichen ein Gemisch der dem eingesetzten Amin entsprechenden Carbamidsäure-(2-phenoxy-ethyl)-ester dar, wie ein infrarotspektroskopischer Vergleich mit einer authentischen, auf anderem Weg aus demselben Amin hergestellten Substanz zeigte. Im Destillat wurden insgesamt 348 g Anilin nachgewiesen.

**Patentansprüche**

1. Verfahren zur Herstellung von N-substituierten Urethanen der allgemeinen Formel

$R^1(NH-CO-OR^2)_n$,

in welcher

$R^1$ für einen gesättigten, unsubstituierten, aliphatischen Kohlenwasserstoffrest mit 4 bis 18 Kohlenstoffatomen, einen gesättigten, gegebenenfalls alkylsubstituierten und/oder Methylenbrücken aufweisenden cycloaliphatischen Kohlenwasserstoffrest mit insgesamt 6 bis 25 Kohlenstoffatomen oder einen gegebenenfalls alkyl- und/oder halogen-substituierten und/oder Methylenbrükken aufweisenden aromatischen Kohlenwasserstoffrest mit insgesamt 6 bis 25 Kohlenstoffatomen steht,

$R^2$ die Bedeutung eines gegebenenfalls inerte Substituenten und/oder Ethergruppen aufweisenden aliphatischen Kohlenwasserstoffrestes mit insgesamt 6 bis 18 Kohlenstoffatomen, eines gegebenenfalls inerte Substituenten aufweisenden cycloaliphatischen Kohlenwasserstoffrestes mit insgesamt 6 bis 15 Kohlenstoffatomen oder eines gegebenenfalls inerte Substituenten aufweisenden araliphatischen Kohlenwasserstoffrestes mit insgesamt 7 bis 18 Kohlenstoffatomen hat, und

n für 1, 2 oder 3 steht,

dadurch gekennzeichnet, daß man

a) primäre Amine der allgemeinen Formel

$R^1(NH_2)_n$,

in der $R^1$ und n die genannte Bedeutung haben, mit

b) Harnstoffen der allgemeinen Formel

$R^3-NH-CO-NH-R^3$,

in welcher $R^3$ die Bedeutung eines einem gegebenenfalls inerte Substituenten aufweisenden primären aromatischen Monoamin mit einem bei Normaldruck mindestens 10°C unterhalb des Siedepunktes des Amins $R^1(NH_2)_n$ liegenden Siedepunkt zugrundeliegenden, gegebenenfalls inerte Substituenten aufweisenden, aromatischen Kohlenwasserstoffrestes mit insgesamt 6 bis 14 Kohlenstoffatomen hat, und

c) primären oder sekundären Alkoholen, die bei Normaldruck einen mindestens 5°C höheren Siedepunkt als das Amin $R^3-NH_2$ besitzen und der allgemeinen Formel

$R^2-OH$

entsprechen, in der $R^2$ die genannte Bedeutung hat,

bei Temperaturen zwischen 150 und 300°C umsetzt und entstehendes Amin $R^3-NH_2$, gegebenenfalls im Gemisch mit Alkohol $R^2-OH$, während der Umsetzung destillativ aus dem Reaktionsgemisch entfernt.

2. Verfahren nach Anspruch 1, dadurch gekennzeichnet, daß man als Ausgangsmaterial b) für die Umsetzung rohe Diarylharnstoffe $R^3-NH-CO-NH-R^3$ verwendet, die man in einer vorgeschalteten Reaktion dadurch erhält, daß man Harnstoff mit Arylaminen $R^3-NH_2$ im Molverhältnis 1 : 1,8 bis 1 : 10 bei Temperaturen zwischen 130 und 250°C unter Abspaltung und Abtrennung von Ammoniak umsetzt.

3. Verfahren nach Ansprüchen 1 und 2, dadurch gekennzeichnet, daß man als Diarylharnstoff $R^3-NH-CO-NH-R^3$ N,N'-Diphenylharnstoff verwendet.

**Claims**

1. Process for the preparation of N-substituted urethanes of the general formula

$R^1(NH-CO-OR^2)_n$

in which

$R^1$ represents a saturated, unsubstituted, aliphatic hydrocarbon radical with 4 to 18 carbon atoms, a saturated cycloaliphatic hydrocarbon radical which has a total of 6 to 25 carbon atoms and is

optionally alkyl-substituted and/or contains methylene bridges, or an aromatic hydrocarbon radical which has a total of 6 to 25 carbon atoms and is optionally alkyl-substituted and/or halogen-substituted and/or contains methylene bridges,

$R^2$ denotes an aliphatic hydrocarbon radical which has a total of 6 to 18 carbon atoms and optionally contains inert substituents and/or ether groups, a cycloaliphatic hydrocarbon radical which has a total of 6 to 15 carbon atoms and optionally contains inert substituents, or an araliphatic hydrocarbon radical which has a total of 7 to 18 carbon atoms and optionally contains inert substituents, and

n represents 1, 2 or 3,

characterised in that

a) primary amines of the general formula

$RX^1(NH_2)_n$

in which $R^1$ and n have the stated meaning, are reacted with

b) ureas of the general formula

$R^3-NH-CO-NH-R^3$

in which $R^3$ denotes an aromatic hydrocarbon radical which has a total of 6 to 14 carbon atoms, optionally contains inert substituents and constitutes the basis of a primary aromatic monoamine which optionally contains inert substituents and has a boiling point at normal pressure at least 10° C below the boiling point of the amine $R^1(NH_2)_n$, and

c) primary or secondary alcohols which have at normal pressure a boiling point at least 5° C higher than the amine $R^3-NH_2$ and which correspond to the general formula

$R^2-OH$

in which $R^2$ has the stated meaning,

at temperatures between 150 and 300'' C and the amine $R^3-NH_2$ formed, optionally in admixture with alcohol $R^2-OH$, is removed, by distillation, from the reaction mixture during the reaction.

2. Process according to Claim 1, characterised in that the starting material b) used for the reaction comprises crude diarylureas $R^3-NH-CO-NH-R^3$ which are obtained in a foregoing reaction by reacting urea with arylamines $R^3-NH_2$ in a molar ratio of 1 : 1,8 to 1 : 10 at temperatures between 130 and 250° C with the liberation and removal of ammonia.

3. Process according to Claims 1 and 2, characterised in that N,N'-diphenylurea is used as the diarylurea $R^3-NH-CO-NH-R^3$.

**Revendications**

1. Procédé de préparation d'uréthannes substitués à l'azote, de formule générale:

$R^1(NH-CO-OR^2)_n$

dans laquelle

$R^1$ représente un reste d'hydrocarbure aliphatique non substitué saturé comportant 4 à 18 atomes de carbone, un reste d'hydrocarbure cycloaliphatique saturé, éventuellement substitué par un groupe alkyle et/ou présentant un pont méthylène, et ayant au total 6 à 25 atomes de carbone, ou un reste d'hydrocarbure aromatique éventuellement substitué par un groupe alkyle et/ou par de l'halogène et/ou présentant un pont méthylène, et ayant au total 6 à 25 atomes de carbone,

$R^2$ a le sens d'un reste d'hydrocarbure aliphatique, ayant au total 6 à 18 atomes de carbone et présentant éventuellement des substituants inertes et/ou des groupes éthers, d'un reste d'hydrocarbure cycloaliphatique présentant éventuellement des substituants inertes ayant au total 6 à 15 atomes de carbone ou d'un reste d'hydrocarbure araliphatique présentant éventuellement des substituants inertes ayant au total 7 à 18 atomes de carbone, et

n vaut 1, 2 ou 3,

procédé caractérisé en ce que l'on fait réagir à des températures comprises entre 150 et 300° C:

a) des amines primaires de formule générale:

$R^1(NH_2)_n$

dans laquelle $R^1$ et n ont le sens indiqué, avec

b) des urées de formule générale:

$R^3-NH-CO-NH-R^3$

dans laquelle $R^3$ a le sens d'un reste d'hydrocarbure aromatique, comportant au total 6 à 14 atomes de carbone, présentant éventuellement les substituants inertes et se trouvant à la base d'une monoamine aromatique primaire, présentant éventuellement des substituants inertes et ayant sous la pression normale un point d'ébullition se situant à au moins 10° C au-dessous du point l'ébullition de l'amine $R^1(NH_2)_n$, et

c) des alcools primaires ou secondaires qui ont sous la pression normale un point l'ébullition supérieur d'au moins 5° C à celui de l'amine $R^3-NH_2$, et qui répondent à la formule générale:

$R^2-OH$

dans laquelle $R^2$ a le sens indiqué,

et l'on élimine du mélange réactionnel, par distillation pendant la réaction, l'amine $R^3-NH_2$ obtenue, éventuellement en mélange avec l'alcool $R^2-OH$.

2. Procédé selon la revendication 1, caractérisé en ce qu'on utilise comme matière de départ b) pour la réaction des diarylurées brutes $R^3-NH-CO-NH-R^3$, que l'on obtient dans une réaction préliminaire en faisant réagir l'urée avec des arylamines $R^3-NH_2$ selon un rapport molaire de 1 : 1,8 à 1 : 10 à des températures comprises entre 130 et 250° C avec scission et séparation d'ammoniac.

3. Procédé selon les revendications 1 et 2, caractérisé en ce qu'on utilise comme diarylurée $R^3-NH-CO-NH-R^3$ la N,N'-diphénylurée.